# EUROPÄISCHE PATENTANMELDUNG

(11) **EP 2 065 071 A1**
(43) Veröffentlichungstag der Anmeldung: **03.06.2009**
(21) Anmeldenummer: 08167840.1
(22) Anmeldetag: 29.10.2008
(51) Int. Cl.: A61N 1/05, A61N 1/365

(54) **Intrakardiale Elektrodenleitung und Herzstimulator**

(30) Priorität: 28.11.2007 DE 102007057227
(71) Anmelder: BIOTRONIK CRM Patent AG, 6341 Baar (CH)
(72) Erfinder: Dörr, Thomas, 12437, Berlin (DE)
(74) Vertreter: Lindner-Vogt, Karin L.

(57) **Zusammenfassung**

Die Erfindung betrifft eine intrakardial implantierare Elektrodenleitung zum Anschluss an ein implantierbares medizinisches Gerät, insbesondere einen Herzschrittmacher oder Kardioverter/Defibrillator oder dergleichen, die im Bereich ihres distalen Endes einen Beschleunigungssensor aufweist, der zur Aufnahme und Unterscheidung von Beschleunigungswerten in wenigstens zwei unterschiedlichen Richtungen ausgebildet ist.

Außerdem betrifft die Erfindung eine Herzstimulationsanordnung, die neben einer solchen Elektrodenleitung auch einen Herzstimulator aufweist.

## Beschreibung

Die Erfindung betrifft eine Elektrodenleitung für einen implantierbaren Herzstimulator für Abgabe elektrischer Stimulationsimpulse zur Therapie eines Herzens, z.B. die kardiale Resynchronisationstherapie (CRT) eines Herzens, sowie einen solchen Herzstimulator selbst. Der Herzstimulator kann ein Herzschrittmacher oder ein implantierbarer Kardioverter/Defibrillator (ICD) oder eine Kombination aus beidem sein, der in der Lage ist, beide Ventrikel eines Herzens zu stimulieren.

Typischerweise weist ein derartiger Herzstimulator wenigstens eine rechtsventrikuläre Sensingeinheit und eine rechtsventrikuläre Stimulationseinheit sowie eine linksventrikuläre Sensingeinheit und eine linksventrikuläre Stimulationseinheit auf. Diese Einheiten sind im Betrieb des Herzstimulators jeweils über Elektrodenleitungen mit an geeigneten Stellen im Herzen zu implantierenden Elektroden verbunden. Die Elektrodenleitung mit den Elektroden zum Erfassen elektrischer Potentiale im linken Ventrikel des Herzens sowie zur Abgabe linksventrikulärer Stimulationsimpulse sind typischerweise Bestandteil einer linksventrikulären Elektrodenleitung, die durch den Koronarsinus eines Herzens verlegt wird und daher auch als Koronarsinuselektrodenleitung bezeichnet wird. Die Elektroden zum Erfassen elektrischer Potentiale im rechten Ventrikel sowie zur Abgabe rechtsventrikulärer Stimulationsimpulse sind typischerweise an einer rechtsventrikulären Elektrodenleitung befestigt, deren distales Ende bis in den Apex des rechten Ventrikels ragt. Die Elektrodenleitungen sind typischerweise an ihrem proximalen Ende über normierte Steckverbindungen mit einem entsprechenden Herzstimulator verbunden.

Die typischen Stimulationsmodi eines rechtsventrikulären Herzstimulators, wie beispielsweise VVI, VVD oder DDD, können als bekannt vorausgesetzt werden. Gleiches gilt für die Abgabe von Stimulationsimpulsen nur im Bedarfsfall (Demand-Schrittmacher), bei der die Abgabe eines Stimulationsimpulses an eine jeweilige Kammer eines Herzens dann unterbunden wird, wenn zuvor in einem entsprechenden Escape-Intervall eine jeweilige Eigenaktion (intrinsische Kontraktion) der jeweiligen Herzkammer über eine dieser Herzkammer zugeordnete Sensingeinheit des Herzstimulators erfasst wurde. Diese an sich bekannten Konzepte können auch bei dem hier beschriebenen Herzstimulator verwirklicht sein.

Der hier betroffene Herzstimulator ist vorzugsweise ein biventrikulärer Herzstimulator, der grundsätzlich in der Lage ist, beide Ventrikel des Herzens ständig oder bei Bedarf zu stimulieren.

Insbesondre bei derartigen Herzstimulatoren besteht das Bedürfnis, die jeweilige Therapie so gut wie möglich an den jeweiligen hämodynamischen Zustand des Patienten anzupassen.

Derzeit wird an verschiedenen Methoden zur Erfassung der Hämodynamik geforscht. Hier werden entweder direkte Methoden der Druckmessung in Pulmonalvene, linken Atrium oder Ventrikel untersucht oder aber indirekte Methoden der Impedanzkardiographie verfolgt. Die Methoden der Druckmessung haben den Nachteil, dass zusätzliche Sensoren im Herzen implantiert werden. Diese Sensoren erfordern einen erhöhten Aufwand bei der Implantation, da sich die gewünschten Implantationsorte von den Standardelektroden (ICD und Schrittmacher) unterscheiden.

Die impedanzbasierten Verfahren haben den Nachteil, dass hier die hämodynamischen Größen nur indirekt abgeleitet werden können und damit der Nachteil der Nachvollziehbarkeit besteht. Es stellt sich immer die Frage, ob der Arzt an diese Methode glaubt.

Ziel der Erfindung ist es, Informationen für ein elektronisches Implantat über den hämodynamischen Status und dessen Veränderungen in einer deutlich verbesserten und gegenüber bereits bekannten Verfahren stärker korrelierten Art und Weise zu erfassen. Vorzugsweise soll die Erfindung auch das Problem lösen, dass die bislang bekannten Verfahren in Ihrer Wirkung von dem Anwender nicht nachvollzogen werden können und daher häufig Ablehnung finden.

Zur Lösung des erstgenannten Problems ist bereits vorgeschlagen worden, in eine intrakardiale Elektrodenleitung einen Beschleunigungssensor (Akzelerometer) zu integrieren, der Informationen über die Bewegung der Elektrodenleitung liefert, die bis zu einem gewissen Grad mit der Bewegung eines jeweiligen Herzteils korreliert, siehe z.B.

US 2004/0172078, US 2004/0172079 und US 2005/0027320. Weiterhin ist in "An Implantable Intracardiac Accelerometer for Monitoring Myocardial Contractility", Pacing and Clinical Electrophysiology 19 (12), Seiten 2066-2071, eine richtungsunabhängige Anwendung eines in die Elektrode integrierten Akzelerometers beschrieben. Trotz der bereits 1996 publizierten Ergebnisse erfolgt bislang keine erfolgreich kommerzielle Nutzung des Konzeptes.

Der Erfinder ist nun zu der Erkenntnis gelangt, dass ohne eine Richtungsinformation in Bezug auf erfasste Beschleunigungswerte eine Korrelation der wahrzunehmenden Wandbewegung zum Akzelerometersignal (Ausgangssignal des Beschleunigungssensors/ Akzelerometers) nur eingeschränkt möglich ist.

Basierend auf dieser Erkenntnis besteht die Lösung der zuvor genannten Aufgabe in einer Elektrodenleitung mit einem proximalen Ende, welches zum Anschließen der Elektrodenleitung an ein implantierbares medizinisches Gerät, insbesondere einen Herzschrittmacher oder Kardioverter/Defibrillator oder dergleichen ausgebildet ist, sowie einem distalen Ende, welches wenigstens eine Elektrode zur Abgabe von Stimulationsimpulsen oder zur Aufnahme intrakardialer elektrischer Signale trägt, sowie einen Beschleunigungssensor, wobei der Beschleunigungssensor zur Aufnahme von Beschleunigungswerten in wenigstens zwei unterschiedlichen Richtungen ausgebildet ist und somit richtungsabhängige Beschleunigungswerte erfassen und entsprechende Akzelerometerausgangsignale ausgeben kann.

Aufgrund der Richtungsabhängigkeit der erfassten Beschleunigungswerte ist eine spezifische Abbildung einer Wandbewegung einer Herzwand und deren sichere Korrelation zum Cardiac Output gegeben.

Vorzugsweise besitzt die Elektrodenleitung einen Steckkontakt an ihrem proximalen Ende und eine elektrischen Leitung (z.B. in Form eines elektrisch leitenden Drahtes) die eine Kontaktfläche des Steckkontaktes mit der Elektrode elektrisch leitend verbindet. Dabei ist der Beschleunigungssensor mit einem Modulator, und der Modulator wiederum mit der elektrischen Leitung verbunden, wobei der Modulator ausgebildet ist, ein Ausgangssignal des Beschleunigungssensors in modulierter Form auf die elektrische Leitung zu geben. Auf diese Weise können die Akzelerometer-Ausgangssignale ohne weitere elektrische Leitungen in der Elektrodenleitung vorsehen zu müssen drahtgebunden von dem Beschleunigungssensor zu einem ggf. an die Elektrodenleitung angeschlossenen Herzstimulator übertragen werden.

Alternativ hierzu kann der Beschleunigungssensor auch mit einem Sender zum drahtlosen Übertragen der Ausgangssignale des Beschleunigungssensors verbunden sein.

Mit einer derartigen Elektrodenleitung kann eine einen an die Elektrodenleitung angeschlossenen Herzstimulator aufweisende Herzstimulationsanordnung geschaffen werden, die eine auf der tatsächlichen mechanischen Dynamik des Herzmuskels basierenden Information mit direkter Korrelation zur Hämodynamik, die direkt als diagnostische Größe oder aber als Steuergröße für die Therapiesteuerung des elektronischen Implantates genutzt werden kann. Durch die Integration des Sensors in eine ohnehin zu implementierende Elektrode wird die Implantationsprozedur durch diesen zusätzlichen Sensor nicht verändert. Des Weiteren ist bei Integration des Sensors in eine bipolare Sonde keine geänderte Anschlusstechnik für die vorhandenen Implantate nötig (IS-1 Standard).

Vorzugsweise ist die Elektrodenleitung eine linksventrikuläre Elektrodenleitung zur Implantation durch den Coronar Sinus hindurch ausgebildet ist und der Stimulation des linken Ventrikels eines Herzens dient.

Die hier vorgestellte Erfindung besteht aus der Integration eines richtungsabhängigen Beschleunigungs-Sensors in eine intrakardiale Elektrodenleitung, wobei hier die linksventrikuläre Elektrodenleitung (z.B. CS-Elektrodenleitung) deshalb zu bevorzugen ist, weil die linksventrikuläre Dynamik die größte Korrelation mit der Gesamthämodynamik verspricht. Der integrierte Beschleunigungssensor liefert dabei neben der Beschleunigungsinformation auch die Richtung der Beschleunigung in mindestens 2 Vektoren.

Der mit dieser Elektrodenleitung im Betrieb verbundene Herzstimulator oder implantierbare Monitor soll neben den geläufigen intrakardialen Elektrogrammsignalen zusätzlich das Akzelerometer-Ausgangssignal des in die Elektrode integrierten richtungsabhängigen Beschleunigungssensors auswerten und mindestens als diagnostische Information oder auch als Steuersignal für die Therapie des elektronischen Implantates einer Auswerte- und Steuereinheit zur Verfügung stellen.

Um in Verbindung mit der Echokardiographie eine einfache Korrelation der Akzelerometer-Ausgangssignale zur Hämodynamik des Patienten zu ermöglichen, weist die Elektrodenleitung gemäß einer bevorzugten Ausführungsvariante im Bereich Ihres distalen Endes wenigstens einen radioopaken oder echodichten Abschnitt auf, der mittels bildgebender Verfahren wie der Computertomographie oder eben der Echokardiographie gut erkennbar ist. Durch zusätzliches Einbringen echodichter Strukturen in die Elektrodenleitung ergibt sich deren Sichtbarkeit in der Echokardiographie, so dass die Signale des Beschleunigungssensors mit den aus der Stress- / Echokardiographie gewonnenen hämodynamischen Befunden korreliert werden können.

Besonders bevorzugt ist eine Herzstimulationsanordnung mit einer linksventrikulären Elektrodenleitung der zuvor bschriebenen Art sowie einem implantierbaren Herzstimulator, an den die linksventrikuläre Elektrodenleitung sowie eine rechtsventrikuläre Elektrodenleitung angeschlossen sind. Hierbei trägt auch die rechtsventrikuläre Elektrodenleitung im Bereich Ihres distalen Endes einen rechtsventrikulären Beschleunigungssensor, der zur Aufnahme von Beschleunigungswerten in wenigstens zwei unterschiedlichen Richtungen ausgebildet ist.

Bei einer derartigen Anordnung mit zwei Elektrodenleitungen mit Beschleunigungssensoren an ihren distalen Enden gestattet die richtungsbezogene Beschleunigungsinformation eine sichere Abstandsmessung zweier implantierter Sonden. Ohne die Richtungsinformation könnten gegenläufige Bewegungen nicht von gleichgerichteten Bewegungen differenziert werden. Die Akzelerometer-Ausgangssignale der beiden in den unterschiedlichen Elektrodenleitung angeordneten Beschleunigungssensoren erlauben es zunächst, die Abstandsänderung der distalen Enden der Elektrodenleitungen aus den erfassten Beschleunigungswerten zu berechnen. Diese ist hinreichend, um kurzfristige Änderungen der Haemodynamik zu erfassen. Für eine langfristige Auswertung kann zusätzlich eine Kalibration dieses Systems z.B. mit Hilfe der Echokardiographie erfolgen. Ebenso kann ein Vergleich mit Referenzkurven (siehe auch weiter unten), die zu definierten Phasen (z.B. in Ruhe) aufgenommen wurden, erfolgen.

Auch die rechtsventrikuläre Elektrodenleitung besitzt einen Steckkontakt an ihrem proximalen Ende und eine elektrische Leitung, die eine Kontaktfläche des Steckkontaktes mit einer Elektrode im Bereich des distalen Endes der rechtsventrikulären Elektrodenleitung elektrisch leitend verbindet. Auch der rechtsventrikuläre Beschleunigungssensor ist vorzugsweise mit einem Modulator verbunden, der seinerseits mit der ohnehin schon vorhandenen elektrischen Leitungen verbunden ist, so dass keine zusätzlichen Leitungen erforderlich sind. Dies ist möglich, weil der Modulator ein jeweiliges Akzelerometer-Ausgangssignal des Beschleunigungssensors in modulierter Form auf die elektrische Leitung gibt, so dass dieses zusätzlich zu eventuellen Stimulationsimpulsen über die gleiche Leitung (bzw. das gleiche Leitungspaar) zu übertragen ist.

Anstelle des Modulators oder zusätzlich zu diesem kann auch ein Sender zum drahtlosen Übertragen der Ausgangssignale des Beschleunigungssensors mit dem rechtsventrikulären Beschleunigungssensor verbunden sein.

Vorzugsweise ist in ein Gehäuse des Herzstimulators ein weiterer Beschleunigungssensor integriert. Dann kann eine Kompensation der überlagerten Bewegung des Patienten mittels Auswertung der Akzelerometer-Ausgangssignale des zusätzlich in dem Gehäuse des Herzstimulators integrierten Beschleunigungssensors erfolgen.

Der Herzstimulator weist darüber hinaus vorzugsweise eine Auswerteeinheit auf, die wenigstens indirekt mit dem Beschleunigungssensor verbunden oder zu verbinden ist und die ausgebildet ist, ein jeweiliges Akzelerometer-Ausgangssignal durch einen Vergleich mit in dem Herzstimulator gespeicherten Referenzkurven auszuwerten, wobei die gespeicherten Referenzkurven unter definierten Bedingungen gewonnen wurden, z.B. in Ruhe oder durch den Arzt im Rahmen einer Echokardiographieuntersuchung.

Weitere vorteilhafte Ausgestaltungen ergeben sich durch Kombination der hier beschriebenen Merkmale untereinander und mit solchen Merkmalen, die aus dem Stand der Technik bekannt sind.

Die Erfindung soll nun anhand eines Ausführungsbeispiels unter Bezug auf die Figuren näher erläutert werden. Diese zeigen in
- Figur 1:: eine schematische Darstellung eines Herztherapiesystems;
- Figur 2:: eine Darstellung eines Herzstimulators mit angeschlossenen, im Herzen angeordneten Elektroden;

- Figur 3:: ein schematisches Blockschaltbild eines Herzstimulators;
- Figur 4:: ein schematisches Blockschaltbild zur näheren Erläuterung des erfindungsgemäßen Herzstimulators vor dem Hintergrund des Blockschaltbildes aus Figur 3;
- Figur 5:: einen Längsschnitt durch das distale Ende einer per Stilett zu implantierenden Elektrodenleitung mit Beschleunigungssensor; und
- Figur 6:: einen Längsschnitt durch das distale Ende einer per Stilett zu implantierenden Elektrodenleitung mit Beschleunigungssensor.

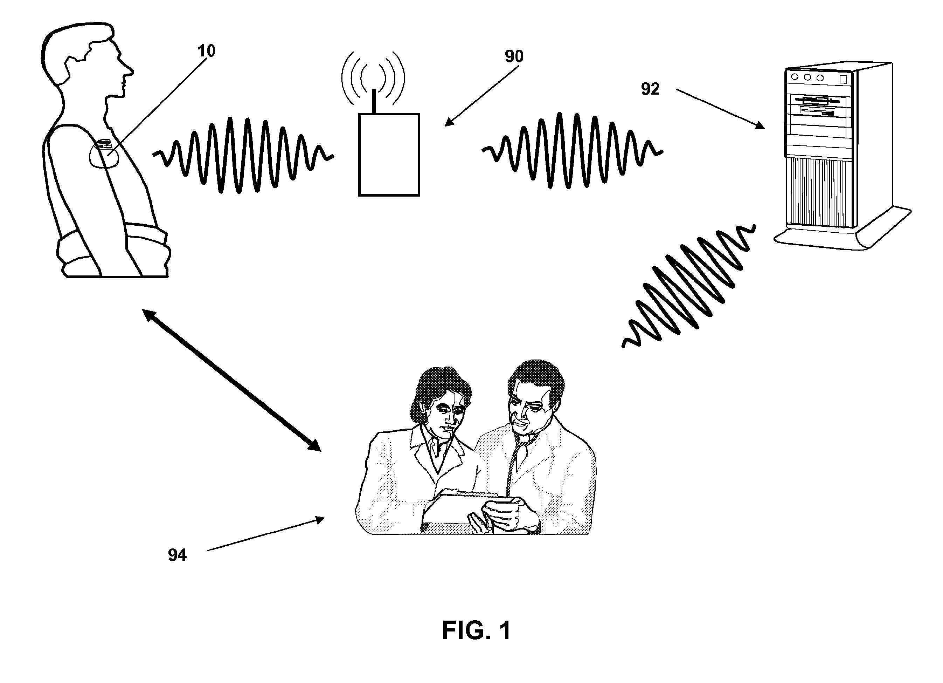

In Figur 1 ist zur Übersicht ein Herztherapiesystem dargestellt, dass neben einem implantierten Herzschrittmacher 10 ein externes Gerät (Patientengerät) 90 sowie ein durch einen Server symbolisch dargestelltes Servicecenter 110 umfasst. Der implantierbare Herzstimulator 10 besitzt eine Telemetrieeinheit, für die er drahtlos Daten mit dem externen Gerät 90 austauschen kann. Das externe Gerät 90 ist beispielsweise drahtgebunden mit dem Servicecenter 92 verbunden, so dass insgesamt Daten zwischen dem Servicecenter 92 und dem implantierbaren Herzstimulator 10 über das externe Gerät 90 als Relaisstation ausgetauscht werden können. Ein Ärzteteam 94 kann über einen datentechnischen Zugriff auf das Servicecenter 92 Einsicht in Daten nehmen, die das Servicecenter 92 von dem implantierbaren Herzstimulator 10 erhalten hat.

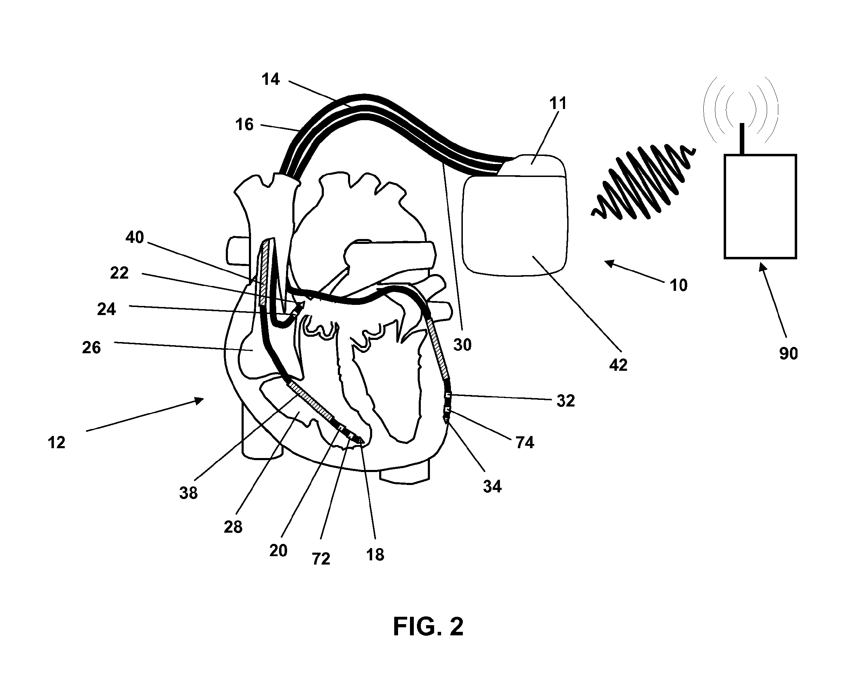

Figur 2 zeigt den implantierbaren Herzstimulator 10 in Form eines Dreikammerherzschrittmachers/Kardioverter/Defibrillators mit daran angeschlossenen Elektrodenleitungen 14, 16 und 30 in Verbindung mit einem Herz 12. Außerdem ist noch einmal das externe Gerät 90 in der Nähe des implantierbaren Herzstimulators 10 dargestellt. Die Elektrodenleitungen 14, 16 und 30 sind über bekannte, standardisierte Steckverbindungen mit Kontaktbuchsen in einem Header (Anschlussgehäuse) 11 des Herzstimulators 10 elektrisch verbunden. Auf diese Weise sind die Elektrodenleitungen 14, 16 und 30 auch mit elektronischen Komponenten im inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 14 ist eine rechtsatriale Elektrodenleitung und besitzt an ihrem distalen Ende eine atriale Spitzenelektrode RA Tip 22 sowie in kurzer Entfernung davon eine atriale Ringelektrode RA Ring 24, die beide im rechten Atrium 26 des Herzens 12 platziert sind.

Die Elektrodenleitung 16 ist eine rechtsventrikuläre Elektrodenleitung und besitzt an ihrem distalen Ende eine rechtsventrikuläre Spitzenelektrode RV Tip 18 und in unmittelbarer Nähe eine rechtsventrikuläre Ringelektrode RV Ring 20. Beide Elektroden sind im Apex des rechten Ventrikels 28 des Herzens 12 angeordnet.

Außerdem ist die rechtsventrikuläre Elektrodenleitung 16 noch eine rechtsventrikuläre Schockwendel RV Schock 38 als großflächige Elektrode zur Abgabe von Relationsschocks. Eine weitere Schockwendel 40 ist in der Vena Cava Superior angeordnet und wird deshalb nachfolgend auch als SVC-Schockelektrode bezeichnet.

Im Bereich des distalen Endes der rechtsventrikulären Elektrodenleitung 16 ist ein Beschleunigungssensor 72 in die rechtsventrikuläre Elektrodenleitung 16 integriert, der über einen Modulator (nicht dargestellt) mit einer elektrischen Leitung verbunden ist, die auch eine der Elektroden 18 oder 20 mit einer Kontaktfläche eines Anschlusssteckers am proximalen Ende der rechtsventrikulären Elektrodenleitung 16 elektrisch verbindet.

Die Elektrodenleitung 30 ist eine linksventrikuläre Elektrodenleitung an deren distalem Ende eine linksventrikuläre Spitzenelektrode LV Tip 34 sowie in deren Nähe eine linksventrikuläre Ringelektrode LV Ring 32 angeordnet ist. Außerdem trägt die linksventrikuläre Elektrodenleitung 30 eine nicht näher bezeichnete, aber in Figur 2 dargestellte linksventrikuläre Schockwendel zur Abgabe von Defibrillationsschocks an den linken Ventrikel. Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinuselektrodenleitung oder CS-Elektrodenleitung bezeichnet.

Auch in die linksventrikuläre Elektrodenleitung 30 ist ein Beschleunigungssensor 74 integriert, der ebenfalls über einen Modulator (nicht dargestellt) mit einer elektrischen Leitung verbunden ist, die auch eine der Elektroden 32 oder 34 mit einer Kontaktfläche eines Anschlusssteckers am proximalen Ende der linksventrikulären Elektrodenleitung 30 elektrisch verbindet.

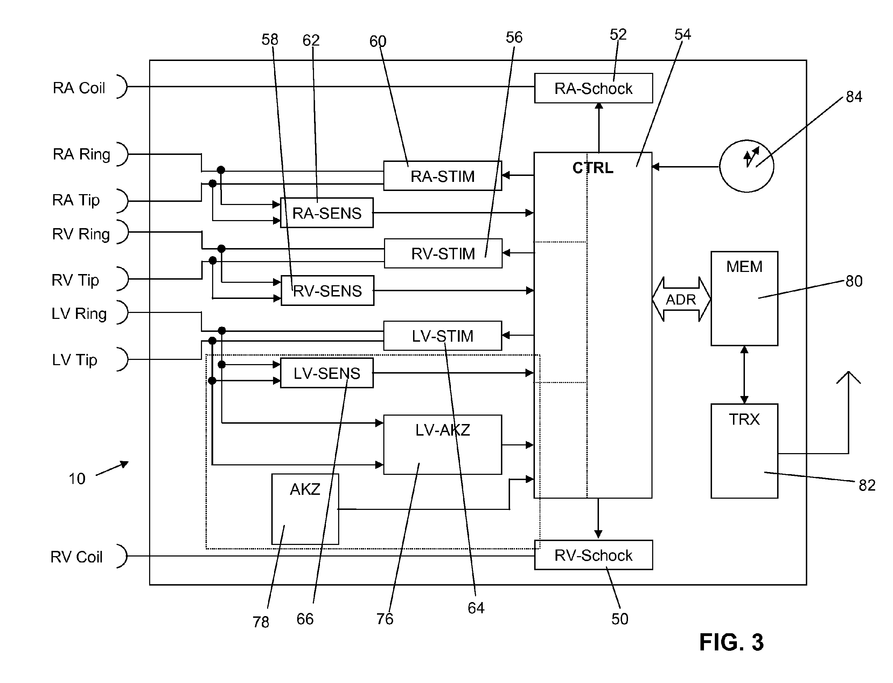

In Figur 3 sind die Hauptbestandteile des Herzstimulators 10 dargestellt. Auf der linken Seite sind die elektrischen Anschlüsse für die verschiedenen Elektroden 18, 20, 22, 24, 32, 34, 38 und 40 dargestellt. Die Schockelektroden 38 und 40 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Tippelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode die rechtsventrikuläre Tippelektrode abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensingeinheit 58 ist dazu ausgebildet, an dem Anschluss für die rechtsventrikuläre Ringelektrode RV Ring und die rechtsventrikuläre Tippelektrode RV Tip anliegende elektrische Potentiale zunächst durch einen Eingangsverstärker zu verstärken und zu filtern. Weiterhin ist die rechtsventrikuläre Sensingeinheit ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R Zacke kennzeichnend für eine natürliche Kontraktion des rechten Ventrikels, die durch Schwellwertvergleich detektiert werden kann. Die rechtsventrikuläre Sensingeinheit 58 gibt daraufhin ein entsprechendes, eine natürliche Kontraktion des rechten Ventrikels anzeigendes Ausgangssignal an die Stimulationssteuereinheit 54 aus.

In analoger Weise sind der Anschluss für die rechtsatriale Tippelektrode und der Anschluss für die rechtsatriale Ringelektrode sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

In gleicher Weise sind auch der Anschluss für die linksventrikuläre Tippelektrode LV Tip und der Anschluss für die linksventrikuläre Ringelektrode LV Ring mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Darüber hinaus sind die Anschlüsse für die linksventrikuläre Tippelektrode LV Tip und die linksventrikuläre Ringelektrode LV Ring mit einer linksventrikulären Akzelerometerauswerteeinheit LV-AKZ 76 verbunden. Die linksventrikuläre Akzelerometerauswerteeinheit 76 ist dazu ausgebildet, dass mit Hilfe des in die linksventrikuläre Elektrodenleitung 30 integrierten Modulators das modulierte Akzelerometerausgangssignal des linksventrikuären Beschleunigungssensors 74 zu verstärken und zu modulieren und schließlich auszuwerten. Die linksventrikuläre Akzelerometerauswerteeinheit 76 erzeugt schließlich ein Ausgangssignal, welches der Steuereinheit CTRL 54 zur weiteren Auswertung zugeführt wird. Das auf diese Weise rückgewonnene Akzelerometer-Ausgangssignal ist richtungsabhängig, d. h. die Größe des Ausgangssignals hängt von der Richtung ab, in die eine Beschleunigungskraft auf den Beschleunigungssensor 74 einwirkt. Der Beschleunigungssensor 74 ist ausgebildet, zwei Akzelerometerausgangssignale zu erzeugen, die jeweils Beschleunigungswerte für zwei verschiedene Richtungen repräsentieren.

In ähnlicher Weise wie für den linksventrikulären Beschleunigungssensor 74 kann auch eine rechtsventrikuläre Akzelerometerauswerteeinheit vorgesehen sein, um Ausgangssignale des rechtsventrikulären Beschleunigungssensors 72 auswerten zu können. Dies ist in Figur 3 hier jedoch nicht dargestellt.

Als weiterer Bestandteil des Herzstimulators 10 ist ein weiterer Beschleunigungssensor 78 mit der Stimulationssteuereinheit 54 verbunden und in das Gehäuse 42 des Herzstimulators 10 integriert. Der Beschleunigungssensor 78 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, bei der Auswertung der Akzelerometer-Ausgangssignale des rechtsventrikulären Beschleunigungssensors 72 und des linksventrikulären Beschleunigungssensors 74 die der Herzeigenbewegung überlagerte Bewegung des Patienten mittels Auswertung der Signale des zusätzlich in dem Gehäuse des elektronischen Implantates integrierten Beschleunigungssensors 78 zu kompensieren.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Die Speichereinheit 80 dient im Rahmen der hier vorgestellten Erfindung insbesondere dazu, Vergleichskurven (Referenzkurven) für die Auswertung der Akzelerometer-Ausgangssignale der Beschleunigungssensoren zu speichern. Diese Vergleichkurven werden unter Zuhilfenahme der Echokardiographie unter vorgegebenen Bedingungen (z.B. Ruhe) aufgenommen und anschließend in der Speichereinheit 80 gespeichert.

Des Weiteren ist die Stimulationssteuereinheit 54 mit einem Zeitgeber 84 verbunden.

Die Speichereinheit 80 ist mit einer Telemetrieeinheit 82 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Als Dreikammerherzstimulator/Kardioverter/Defibrillator ist der Herzstimulator 10 in der Lage, eine Stimulation des rechten Atriums, des rechten Ventrikels und des linken Ventrikels oder auch nur einer oder zweier dieser Herzkammern in an sich bekannter Weise durchzuführen. Dies schließt insbesondere die Stimulation einer jeweiligen Herzkammer im Demand-Modus ein, in der Stimulationsimpulse nur dann an die jeweilige Herzkammer abgegeben werden, falls in einem vorangehenden, jeweiligen Escape-Intervall seitens der jeweiligen Sensingeinheit keine intrinsische Kontraktion der jeweiligen Herzkammer erfasst wird. Damit ist der Herzschrittmacher in der Lage, die bekannten rechtsventrikulären Stimulationsmodi wie VVI, VVD oder DDD durchzuführen.

Für das Timing der Stimulationsimpulse im biventrikulären Stimulationsmodus, in dem beide Ventrikel eines Herzens stimuliert werden, ist insbesondere eine interventrikuläre Verzögerungszeit (VV-Intervall) von Bedeutung, d. h. die Zeit, mit der ein rechter Stimulationsimpuls und ein linker Stimulationsimpuls (sofern sie im Demand-Modus nicht inhibiert werden) aufeinanderfolgen. Diese Zeit kann größer als 0 sein, so dass der linke Stimulationsimpuls dem rechten Stimulationsimpuls nachfolgt. Die interventrikuläre Verzögerungszeit kann 0 sein, was bedeutet, dass ein rechtsventrikulärer Stimulationsimpuls und ein linksventrikulärer Stimulationsimpuls durch gleichzeitige Ansteuerung der rechtsventrikulären Stimulationseinheit 56 in der linksventrikulären Stimulationseinheit 64 durch die Stimulationssteuereinheit 54 gleichzeitig abgegeben werden. Die interventrikuläre Verzögerungszeit kann auch kleiner 0 sein, was bedeutet, dass ein linksventrikulärer Stimulationsimpuls vor der Abgabe des zugehörigen rechtsventrikulären Stimulationsimpulses abgegeben wird.

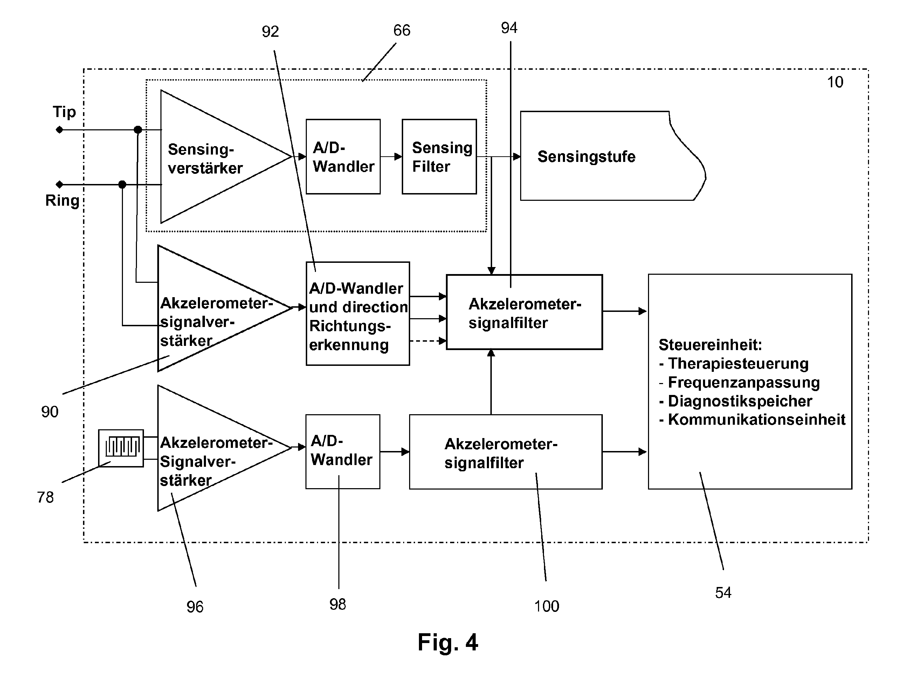

Figur 4 zeigt die Komponenten eines Kanals, in diesem Falle des linksventrikulären Kanals, der in Figur 3 durch eine punktierte Linie angedeutet ist und der sowohl eine linksventrikuläre Sensingeinheit 66 als auch die linksventrikuläre Akzelerometerauswerteeinheit 76 umfasst, in einer detaillierteren Darstellung. In Bezug auf die linksventrikuläre Sensingeinheit 66 ist Figur 4 zu entnehmen, dass diese einen Eingangsverstärker 80, einen Analog-Digital-Wandler 82 und schließlich einen Digitalfilter 86 umfasst, dessen Ausgangswert ein Sensingfunktionsblock der Steuereinheit 54 zugeführt wird.

In Bezug auf die Akzelerometerauswerteeinheit 76 ist Figur 4 zu entnehmen, dass diese ebenfalls einen Signalverstärker 90 umfasst, dessen Ausgangssignal einem Analog-Digital-Wandler und Demodulator 92 zugeführt wird. Der Analog-Digital-Wandler und Demodulator 92 ist insbesondere ausgebildet, aus dem über die Elektrodenleitung empfangenen, modulierten Akzelerometerausgangssignal ein demoduliertes Signal rückzugewinnen und insbesondere zwei verschiedene Ausgangssignale für zwei unterschiedliche Beschleunigungsrichtungen per Demodulation zu gewinnen. Das Ausgangssignal der Analog-Digital-Wandler- und Demodulatoreinheit 92 wird einem Akzelerometersignalfilter 94 zugeführt und von diesem digital gefiltert. Dem Akzelerometersignalfilter 94 wird ebenso das Ausgangssignal eines Sensingfilters der Sensingeinheit 66 zugeführt. Weiterhin ist der Akzelerometersignalfilter 94 mit dem in das Gehäuse 42 des Herzstimulators 10 integrierten Akzelerometers 72 verbunden, und zwar über einen zweiten Akzelerometersignalverstärker 96, einen daran angeschlossenen Analog-Digital-Wandler 98 und einen zweiten Akzelerometersignalfilter 100.

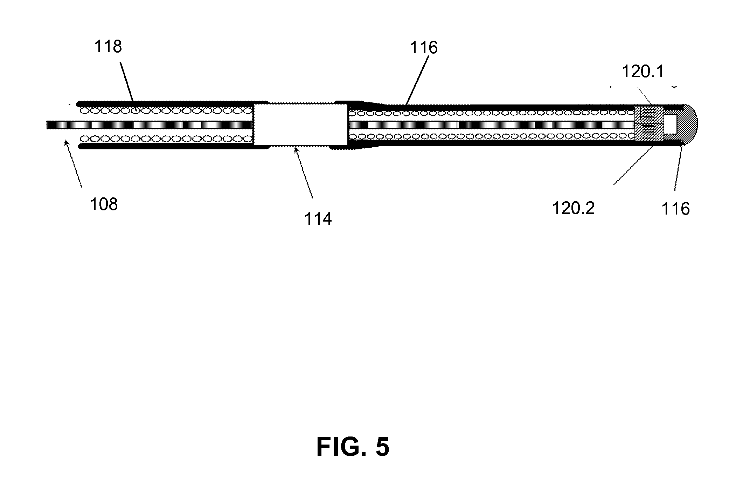

Figur 5 zeigt ein distales Ende einer typischen, mit Hilfe eines Stiletts zu implantierenden Elektrodenleitung 110. Diese besitzt eine Spitzen-Elektrode (Tippelektrode) 112 und eine Ringelektrode 114. Eine flüssigkeitsdichte Hülle 116 dichtet die Elektrodenleitung 110 gegenüber der Umgebung ab. Innerhalb der Hülle 116 ist eine Drahtwendel 118 angeordnet, die der Elektrodenleitung 110 mechanische Stabilität verschafft und gleichzeitig als elektrische Zuleitung dient. Unmittelbar an die Tippelektrode 112 angrenzend ist in die Elektrodenleitung 110 ein doppelter Beschleunigungssensor 120 integriert, der zwei Beschleunigungsaufnehmer 120.1 und 120.2 aufweist, die Beschleunigungswerte in unterschiedlichen Richtungen aufnehmen. Diese Beschleunigungsaufnehmer 120.1 und 120.2 sind über einen nicht dargestellten Modulator mit der Drahtwendel 118 der Elektrodenleitung 110 elektrisch verbunden und können auf diese Weise ein Ausgangssignal des Beschleunigungssensors 120 modulieren und über die Drahtwendel 118 an einen nicht dargestellten Kontakt eines Elektrodenleitungssteckers übertragen.

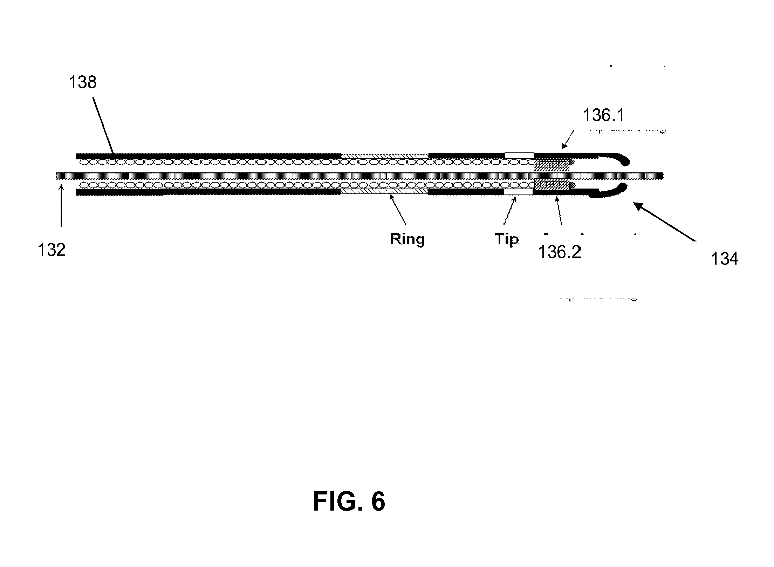

Figur 6 zeigt einen Längsschnitt durch das distale Ende einer alternativen Elektrodenleitung 130. Diese unterscheidet sich von der Elektrodenleitung 110 dadurch, dass sie mit Hilfe eines Führungsdrahtes 132 implantierbar ist und hierzu ein offenes distales Ende 134 aufweist. Im Bereich dieses offenen distalen Endes 134 ist wiederum ein Beschleunigungssensor 136 mit zwei Beschleunigungsaufnehmern 136.1 und 136.2 angeordnet, die jeweils Beschleunigungswerte für unterschiedliche Beschleunigungsrichtungen liefern. Auch diese beiden Beschleunigungsaufnehmer 136.1 und 136.2 sind über einen nicht dargestellten Modulator mit einer Drahtwendel 138 im Inneren der Elektrodenleitung 130 verbunden.

### Bezugszeichenliste

| Bezugszeichen | Bedeutung |
|---|---|
| 10 | Herzstimulator |
| 11 | Header (Anschlussgehäuse) |
| 12 | Herz |
| 14 | rechtsatriale Elektrodenleitung |
| 16 | rechtsventrikuläre Elektrodenleitung |
| 18 | rechtsventrikuläre Spitzenelektrode RV Tip |
| 20 | rechtsventrikuläre Ringelektrode RV Ring |
| 22 | atriale Spitzenelektrode RA Tip |
| 24 | atriale Ringelektrode RA Ring |
| 26 | rechtes Atrium |
| 28 | rechter Ventrikel |
| 30 | linksventrikuläre Elektrodenleitung |
| 32 | linksventrikuläre Ringelektrode LV Ring |
| 34 | linksventrikuläre Spitzenelektrode LV Tip |
| 38 | rechtsventrikuläre Schockwendel RV Schock |
| 40 | Schockwendel |
| 42 | Gehäuse |
| 50 | rechtsventrikulärer Schockimpulsgenerator |
| 52 | SVC-Schockimpulsgenerator |
| 54 | Stimulationssteuereinheit |
| 56 | rechtsventrikuläre Stimulationseinheit |
| 58 | rechtsventrikuläre Sensingeinheit |
| 60 | rechtsatriale Stimulationseinheit |
| 62 | rechtsatriale Sensingeinheit |
| 64 | linksventrikuläre Stimulationseinheit |
| 66 | linksventrikuläre Sensingeinheit |
| 72 | rechtsventrikulärer Beschleunigungssensor |
| 74 | linksventrikulärer Beschleunigungssensor |
| 76 | Akzelerometerauswerteeinheit |
| 78 | ins Gehäuse integrierter Beschleunigungssensor |
| 80 | Speichereinheit |
| 82 | Telemetrieeinheit |
| 84 | Zeitgeber |
| 86 | externes Gerät |
| 88 | Servicecenter |
| 89 | Ärzteteam |
| 90 | Signalverstärker |
| 92 | Demodulator |
| 94 | Akzelerometersignalfilter |
| 96 | Akzelerometersignalverstärker |
| 98 | Analog-Digitalwandler |
| 100 | Zweiter Akzelerometersignalfilter |
| 108 | Stilett |
| 110 | Stilett-implantierbare Elektrodenleitung (distales Ende) |
| 112 | Tip-Elektrode |
| 114 | Ring-Elektrode |
| 116 | Hülle |
| 118 | Drahtwendel |
| 120 | Beschleunigungssensor |
| 130 | Over-the-wire Elektrodenleitung (distales Ende) |
| 132 | Führungsdraht |
| 134 | Offenes distales Ende |
| 136 | Beschleunigungssensor |
| 138 | Drahtwendel |

## Patentansprüche

1. Elektrodenleitung mit einem proximalen Ende, welches zum Anschließen der Elektrodenleitung an ein implantierbares medizinisches Gerät, insbesondere einen Herzschrittmacher oder Kardioverter/Defibrillator oder dergleichen ausgebildet ist, sowie einem distalen Ende, welches wenigstens eine Elektrode zur Abgabe von Stimulationsimpulsen oder zur Aufnahme intrakardialer elektrischer Signale trägt, sowie einen Beschleunigungssensor, **dadurch gekennzeichnet, dass** der Beschleunigungssensor zur Aufnahme und Unterscheidung von Beschleunigungswerten in wenigstens zwei unterschiedlichen Richtungen ausgebildet ist.

2. Elektrodenleitung nach Anspruch 1, mit einem Steckkontakt an ihrem proximalen Ende und einer elektrischen Leitung (leitender Draht) die eine Kontaktfläche des Steckkontaktes mit der Elektrode elektrisch leitend verbindet, **dadurch gekennzeichnet, dass** der Beschleunigungssensor mit einem Modulator, und der Modulator mit der elektrischen Leitung verbunden ist, wobei der Modulator ausgebildet ist, ein Ausgangssignal des Beschleunigungssensors in modulierter Form auf die elektrische Leitung zu geben.

3. Elektrodenleitung nach Anspruch 1, **dadurch gekennzeichnet, dass** der Beschleunigungssensor mit einem Sender zum drahtlosen Übertragen der Ausgangssignale des Beschleunigungssensors verbunden ist.

4. Elektrodenleitung nach einem der Ansprüche 1 bis 3, **dadurch gekennzeichnet, dass** die Elektrodenleitung zur Implantation durch den Coronar Sinus hindurch ausgebildet ist.

5. Elektrodenleitung nach einem der Ansprüche 1 bis 4, **dadurch gekennzeichnet, dass** die Elektrodenleitung im Bereich Ihres distalen Endes wenigstens einen radioopaken Abschnitt aufweist, der mittels bildgebender Verfahren wie der Computertomographie gut erkennbar ist.

6. Herzstimulationsanordnung mit einer Elektrodenleitung gemäß einem der Ansprüche 1 bis 5 sowie einem implantierbaren Herzstimulator mit einem Gehäuse **dadurch gekennzeichnet, dass** in das Gehäuse des Herzstimulators ein weiterer Beschleunigungssensor integriert ist.

7. Herzstimulationsanordnung mit einer Elektrodenleitung gemäß einem der Ansprüche 1 bis 5 sowie einem implantierbaren Herzstimulator **dadurch gekennzeichnet, dass das** der Herzstimulator eine Auswerteeinheit aufweist, die wenigstens indirekt mit dem Beschleunigungssensor verbunden und ausgebildet ist, ein jeweiliges Akzelerometer-Ausgangssignal durch einen Vergleich mit in dem Herzstimulator gespeicherten Referenzkurven auszuwerten, wobei die gespeicherten Referenzkurven unter definierten Bedingungen gewonnen wurden.

8. Herzstimulationsanordnung mit einer linksventrikulären Elektrodenleitung gemäß Anspruch 4 sowie einem implantierbaren Herzstimulator, an den die linksventrikuläre Elektrodenleitung sowie eine rechtsventrikuläre Elektrodenleitung angeschlossen sind, **dadurch gekennzeichnet, dass** die rechtsventrikuläre Elektrodenleitung im Bereich Ihres distalen Endes einen rechtsventrikulären Beschleunigungssensor trägt, der zur Aufnahme von Beschleunigungswerten in wenigstens zwei unterschiedlichen Richtungen ausgebildet ist.

9. Herzstimulationsanordnung nach Anspruch 9, deren rechtsventrikuläre Elektrodenleitung einen Steckkontakt an ihrem proximalen Ende und eine elektrische Leitung (leitender Draht), die eine Kontaktfläche des Steckkontaktes mit einer Elektrode im Bereich des distalen Endes der rechtsventrikulären Elektrodenleitung elektrisch leitend verbindet, aufweist, **dadurch gekennzeichnet, dass** der rechtsventrikuläre Beschleunigungssensor mit einem Modulator, und der Modulator mit den elektrischen Leitungen verbunden ist, wobei der Modulator ausgebildet ist, ein Ausgangssignal des Beschleunigungssensors in modulierter Form auf die elektrische Leitung zu geben.

10. Herzstimulationsanordnung nach Anspruch 8, **dadurch gekennzeichnet, dass** der rechtsventrikuläre Beschleunigungssensor mit einem Sender zum drahtlosen Übertragen der Ausgangssignale des Beschleunigungssensors verbunden ist.
